# EUROPEAN PATENT SPECIFICATION

(11) **EP 1 030 686 B1**
(45) Date of publication and mention of the grant of the patent: **17.03.2004**
(21) Application number: 98903356.8
(22) Date of filing: 28.01.1998
(51) Int. Cl.: A61K 41/00

(54) **LYOPHILIZATE OF LIPID COMPLEX OF WATER INSOLUBLE PORPHYRINS**
LIPIDKOMPLEX-LYOPHILISAT VON WASSERUNLÖSLICHEN PORPHYRINEN
LYOPHILISAT DE COMPLEXE LIPIDIQUE DE PORPHYRINES INSOLUBLES DANS L'EAU

(30) Priority: 28.01.1997 US 789566
(43) Date of publication of application: 30.08.2000
(73) Proprietor: PHARMACIA & UPJOHN COMPANY, Kalamazoo, Michigan 49001 (US)
(72) Inventor: CHERIAN, Mathew, I-20020 Arese (IT); APTE, Shireesh, Prakash, Bridgewater, NJ 08807 (US)
(74) Representative: Mazzini, Giuseppe
(86) International application number: PCT/US1998/000033
(87) International publication number: WO 1998/032463

(56) References cited:
- EP-A- 0 569 113
- EP-A- 0 720 853
- WO-A-96/32094
- WO-A-97/04746
- US-A- 5 277 913
- US-A- 5 389 378
- US-A- 5 707 608
- KUZELOVA K ET AL: "Interactions of dicarboxylic porphyrins with unilamellar lipidic vesicles: drastic effects of pH and cholesterol on kinetics." BIOCHEMISTRY, SEP 5 1995, 34 (35) P11245-55, UNITED STATES, XP002070076
- MAYHEW E ET AL: "LIPID-ASSOCIATED METHYLPHEOPHORBIDE-A (HEXYL-ETHER) AS A PHOTODYNAMIC AGENT IN TUMOR-BEARING MICE" PHOTOCHEMISTRY AND PHOTOBIOLOGY, vol. 58, no. 6, 1 January 1993, pages 845-851, XP000564985
- GREGORY B W ET AL: "Interfacial complexation of phospholipid Langmuir monolayers with water-soluble porphyrins and phthalocyanines: An X-ray reflectivity study" THIN SOLID FILMS, vol. 284-285, 15 September 1996, page 849-853 XP004078249
- CANNON J B: "PHARMACEUTICS AND DRUG DELIVERY ASPECTS OF HEME AND PORPHYRIN THERAPY" JOURNAL OF PHARMACEUTICAL SCIENCES, vol. 82, no. 5, 1 May 1993, pages 435-446, XP000368106

## Description

### BACKGROUND OF THE INVENTION

The present invention relates to a method for preparing a lyophilizate of a lipid complex of a porphyrin.

Porphyrins are biologically active compounds, usually consisting of four pyrrole rings. Typically, they have a centrally located metal atom and are activated by light radiation of suitable wavelength. Porphyrins display antitumor activity in photodynamic therapy (PDT) where the porphyrins are administered to a patient and localize in neoplastic tissues. Typically, the neoplastic tissues are irradiated with light at a wavelength which corresponds to an absorption band of the porphyrin resulting in the activation of the porphyrin and preferential destruction of the neoplastic tissues. It has been suggested in U.S. Patent 5,162,519 to Bonnett et al. that the mechanism involved is due to the production of the highly reactive singlet oxygen which is produced by transfer of energy from the light-excited porphyrin molecule to an oxygen molecule. Such photodynamic therapy is the subject of a series of articles making up a special issue of Photochemistry and Photobiology, Volume 46, number 5, November, 1987 (hereinafter "P&P 46-5"). According to these articles, photodynamic therapy has been used in the treatment of a wide variety of cancers including such solid tumors as cancers of the bronchial tubes, bladder, esophagus, lung, skin, head and neck, brain, and colon and intraocular and gynecologic cancers.

A major disadvantage to the use of porphyrins in the treatment of cancer is that when used in high concentrations to destroy the tumor cells, the porphyrins exhibit toxic side effects. The porphyrin is generally injected intravenously or intraperitoneally at a dose of about 2 mg per kg of body weight. At lower concentrations which might be more easily tolerated by the patient, the porphyrins tend to exhibit very little effect on the tumor cells.

It has been reported that porphyrins may be incorporated into a liposome and injected intraperitoneally. Spikes et al., "Photodynamic Behavior of Porphyrins in Model Cell, Tissue and Tumor Systems"; in Photodynamic Therapy of Tumors and Other Diseases (Edited by G. Jori and C.A. Perria); pages 45-53; Libreria Progetto, Padua (1985) and references cited therein.

There is a need for compositions containing porphyrins which are effective for the treatment of cancer and yet exhibit less toxic effects towards the patient.

### SUMMARY OF THE INVENTION

It is an object of the present invention to provide a method for preparing a lipid complex of a porphyrin.

The lipid complexes in accordance with this invention appear to be desirable for several reasons. It appears that the lipid complexes may sufficiently slow the release of the porphyrins that the porphyrins can be administered at higher dosage levels with less toxicity thereby improving treatment. The lipid complexes also appear to be more stable to ambient light and UV radiation than the porphyrin alone thus simplifying their handling. While it has not been confirmed, the lipid complexes may also preferentially accumulate in tumor tissues.

While the invention will hereafter be described with respect to the preparation of lipid complexes and lyophilizates of lipid complexes of tin porphyrin, those skilled in the art will appreciate that the methods taught herein are also applicable to the preparation of lipid complexes and lyophilizates of other porphyrins, particularly other metalloporphyrins.

In accordance with one embodiment of the invention, a lyophilizate of a phospholipid-porphyrin complex is prepared by a process comprising the steps of preparing a concentrated solution of a water-insoluble metal porphyrin and a phospholipid in an organic solvent, adding water to the solution to cause formation of a lipid complex of the porphyrin, removing the organic solvent to provide a dispersion of the lipid complex in water as an aqueous phase, dissolving a pharmaceutically acceptable excipient in the aqueous phase of the dispersion, and lyophilizing the dispersion of the lipid complex to form a lyophilizate.

In accordance with a preferred embodiment of the present invention, the lyophilizate is prepared by a method which comprises forming a concentrated solution of tin porphyrin and a mixture of dimyristoyl phosphatidylcholine (DMPC) and dimyristoyl phosphatidylglycerol (DMPG) in chloroform, adding an aqueous solution such as water for injection to form the lipid complex of tin porphyrin and provide a dispersion of the lipid complex in water as an aqueous phase, sparging the dispersion to remove the chloroform, reducing the particle size of the dispersion of the lipid complex, adding an aqueous solution of mannitol as a pharmaceutically acceptable lyophilization excipient to the dispersion, and lyophilizing the composition, wherein a lyophilizate is obtained which upon reconstituting with water provides a colloidal dispersion of a porphyrin-lipid complex. It is possible to prepare the solution of the prophyrin and the phospholipids by dissolving the porphyrin in one organic solvent, dissolving the phospholipids in another organic solvent, mixing the two solutions, and then removing the solvents after addition of water.

### DETAILED DESCRIPTION OF THE INVENTION

The term "water insoluble porphyrin" as used herein means porphyrins having a solubility in water which is less than about 1.5 mg/ml at 23°C and, more typically, is less than 0.5 mg/ml at 23 °C and still more typically less than 0.1 mg/ml at 23°C.

The term "lipid complex" is an art recognized term. Lipid complexes are characterized by a noncovalent bond between the lipid and the porphyrin which is observed by a phase change in differential scanning calorimetry. Liposomes are not lipid complexes within the meaning of the term.

The term "pharmaceutically acceptable aqueous diluent" as used herein refers to water for injection, saline, and other known aqueous vehicles.

The term "lyophilization excipient" refers to a substance which is added to a solution prior to lyophilization to enhance characteristics such as the color, texture, strength, and volume of the cake. Examples of lyophilization excipients are provided below.

The term "photodynamic therapy" (PDT) as used herein refers to a therapy treatment of a patient having a disease such as cancer wherein the patient is treated with a drug such as a porphyrin which has the tendency to preferentially accumulate in neoplastic tissues relative to normal tissues. The drug, when irradiated becomes toxic to the neoplastic tissue.

Porphyrins are biologically active nitrogen-containing compounds having a conjugated cyclic structure consisting of four pyrrole rings linked together through their 2-and 5- positions by methine bridges. The term "porphyrin" includes derivatives wherein a metal atom is chelated into the ring structure, presumably complexed or bonded to at least two of the nitrogens of the pyrrole ring. The metal atom inserted into the ring structure includes, e.g., tin, zinc, lanthanides, actinides, and those metals of the transition series of which chromium, manganese, iron, cobalt, nickel, and copper are exemplary. The preferred porphyrins for use in the present invention are metalloporphyrins, particularly tin porphyrins.

Tin porphyrin and other porphyrins useful in this invention are described in the literature. It is known to irradiate tumors and cancerous tissues in the human body with intensive light following administration of a hematoprophyrin derivative in the wavelength range of 626 to 636 nanometers to reduce and, at times, destroy the cancerous cells (see PCT published specification WO83/00811). It is also known that porphyrins, especially the sodium salt of protoporphyrins, can maintain or promote the normal functions of cells and are useful for preventing the genesis, growth, metastasis, and relapse of malignant tumors. Japanese Published Patent Application No. 125737/76 describes the use of porphyrins as tumor inhibiting agents, exemplifying etioporphyrin, mesoporphyrin, protoporphyrin, deuteroporphyrin, hematoporphyrin, coproporphyrin, and uroporphyrin. U.S. Pat. Nos. 4,837,221 and 5,162,519 to Bonnett et al. teach the therapy of tumors susceptible to necrosis by a porphyrin when adminstered to locate in the tumor and illuminated with light of a wavelength absorbed by the porphyrin. U.S. Pat. No. 5,512,559 to Shalkos et al describes the use of porphyrins and metal complexes of prophyrin in the photodynamic treatment of cancer tumors. European Patent Publication 0 186 962 describes therapy of tumors susceptible to necrosis when pharmacological acceptable meso-porphyrins are administered followed by illumination with light of a wavelength absorbed by the porphyrin.

U.S. Patent 5,407,808, discloses hematoporphyrin derivatives or a mixture of porphyrins derived therefrom (e.g. Phoytofrin II), to mammalian tumor cells and then subject such cells to light of appropriate wavelength to effect cell destruction. Any porphyrin can be used in the present invention provided that it complexes with a phospholipid and is pharmaceutically active. Theoretically, any of these therapies could be modified by a lipid complex of the water insoluble porphyrins and administering it to the patient instead of the uncomplexed porphyrin.In order to produce the lipid complex and administer the complex as a colloidal dispersion in water, the porphyrin must be essentially water insoluble as defined above.

In accordance with the invention the porphyrin, particularly a tin porphyrin, is provided as a concentrated solution of the porphyrin in an organic solvent. The most typical example of the solvent used to prepare this solution is chloroform. However, other organic solvents such as methylene chloride, carbon tetrachloride, ethylene dichloride, freons, DMSO (dimethyl sulfoxide), DMA (dimethyl acetamide), etc. can be used. Useful solvents must form stable solutions with the porphyrin, e.g., the solvent must not interact with, destabilize, or deactivate the drug. In addition, the solubility of the porphyrin in the solvent must also be high enough that the porphyrin can be dissolved in amounts sufficiently high to form commercially useful quantities of the lipid complex, and the solvent should be capable of being removed easily from an aqueous dispersion of the lipid complex as described hereafter. Preferably, a solution having a concentration of about 0.25 to 25 mg/ml, preferably about 2 to 25 mg/ml and most preferably about 15 mg/ml porphyrin is used. The concentration may vary depending upon the nature of the solvent and temperature, but it is advantageous to use a concentrated solution of the porphyrin in preparing the lipid-porphyrin complex. This minimizes the amount of solvent that must be removed later in the process, and it also assists in forcing the porphyrin out of solution and into lipid porphyrin complex formation with the addition of water.

Typically, the phospholipids are dissolved separately. The organic solvent used to prepare the solution of the phospholipids should meet similar requirements to those outlined for the porphyrin solvent. It must be compatible with the phospholipids and not destabilize them or the porphyrins. In addition, the lipids should be soluble enough in the solvent so as to be able to introduce enough of the lipid to form the complex yet minimize the amount of solvent that must be removed later. A solvent which can be readily removed from the dispersion of the lipid complex is most preferred. The solvent most typically used to prepare this solution is chloroform or methylene chloride. Typically the concentration of this phospholipid solution will range from about 10 to 250 mg/ml. Preferably, the same solvent is used to dissolve both the porphyrin and the phospholipid.

Phospholipids are amphophilic in nature, i.e., the molecules have a hydrophobic tail such as a long chain hydrocarbon, and a hydrophilic head. In an aqueous medium, such as water or saline, the tails of the molecules align with each other, away from the aqueous phase, while the heads of the molecules face outward into the aqueous phase. It is this nature of the phospholipids that makes them very useful for formulating soluble compositions from highly insoluble drugs like porphyrins.

The phospholipids used in the invention are preferably selected such that their phase transition temperature is equal to or below the body temperature of about 37°C and the complex releases the drug in the body. Representative examples of useful phospholipids include the synthetic phospholipids, dimyristoyl phosphatidylcholine (DMPC), dimyristoyl phosphatidyl-glycerol (DMPG), dipalmitoylphosphatidylcholine (DPPC), dipalmitoylphosphatidylglycerol (DPPG), distearoylphosphatidycholine (DSPC), distearoylphosphatidylglycerol (DSPG), or a combination thereof. Other examples of phospholipids can be found in the CRC Handbook of Lipid Bilayers by Marsh, M.A., CRC Press (199). When DMPC and DMPG are used in a ratio of DMPC to DMPG of about 7:3 they mimic the cell membrane.

The porphyrin and the phospholipid are added to the solvent such that the weight ratio of the porphyrin to lipid is about 1/10 to 1/100 and preferably about 1/10 to 1/40.

In some applications, it has been found desirable to add cholesterol or its hemisuccinate derivatives or other salt forms to the lipid complex. The cholesterol is believed to slow down the release of the drug. This approach may be particularly desirable with subcutaneous formulations where severe necrosis can result if the drug is delivered too quickly. The cholesterol may be used in amounts up to about 50 parts per hundred parts phospholipid and preferably about 0.5 to 15 parts per 100 parts phospholipid.

Once the lipids and porphyrin have been added to the solvent and thoroughly mixed, water or an aqueous solution is added rapidly to the mixture with stirring for several minutes. Addition of the water is believed to cause the porphyrin and the lipid to come out of solution and complex with each other. The water is preferably added in an amount such that the porphyrin is present in an amount of about 10 mg to 100 mg per 100ml water. It is desirable to limit the amount of water because higher amounts of water increase the amount of water that must be removed during the subsequent lyophilization process. It is believed that complexation may be complete in about 30 minutes. However, it is desirable to stir the dispersion for about one hour to insure that the complexation is complete.

The lipid complex dispersion described above, is treated to remove the solvents. Any of a variety of techniques can be used for this purpose. For example, it has been found that chloroform can be effectively removed if the dispersion is sparged with an inert gas such as nitrogen.

A pharmaceutically acceptable lyophilization excipient is dissolved in the aqueous phase of the dispersion. Mannitol it typically used as the excipient but other excipients which do not interact with the drug or the lipid complex may be used. Sodium or potassium phosphate, citric acid, tartaric acid, gelatin, and carbohydrates such as lactose, dextrose, dextran, hetastarch, etc. are common examples of excipients which are also believed to be useful herein. The excipients can be used alone or in combination to provide a cake of good quality which readily disperses in water upon reconstitution.

The excipients are typically added to the dispersion as solutions in water. Again, it is desirable to use concentrated solutions to minimize the amount of water for removal by lyophilization. The amount of the excipient is adjusted in a manner that is well known in the art to provide a cake which does not crack or shrink and is porous so that it readily dissolves and has a good appearance. Mannitol has been found to be particularly useful. Mannitol is added to the dispersion as a solution having a concentration of about 0.01 to 0.15 g/ml. Mannitol is added in an amount of about 1 to 100 parts by weight per part tin porphyrin.

After removing the solvents and adding the excipient, the dispersion is passed through a homogenizer (e.g., a Tekmar rotor/stator homogenizer, Model T25, or a microfluidics submerged jet homogenizer, Model M110Y). As a general rule, the smaller the particle size of the dispersion, the faster the formulation can be dried during the lyophilization cycle. A dispersion having a particle size distribution ranging from about 10 to 4000 nm and averaging about 600 nm has been found to be satisfactory for lyophilization. The optimum particle size may vary depending on the mode of administration.

A typical lyophilization cycle useful in accordance with the present invention is provided below. The cycle may be varied depending upon the equipment and facilities available in a manner well known in the art.

The homogenized formulation can be poured into vials of a 5 to 50mL nominal volume. The vials are placed into a lyophilization chamber at about 5 °C. The vial size will usually be selected such that each vial contains a single dosage of the porphyrin-lipid complex. The temperature of the chamber is reduced to -30°C over a period of one hour after which the temperature is maintained at -30°C for about four hours. The pressure in the lyophilization chamber is then reduced to 200-250 microns of pressure for the remainder of the cycle. After reducing the pressure in the chamber, the temperature is ramped up to +25C over a period of 15 hours and the product is held at +25°C for five hours. The temperature then is ramped up to +40°C over a period of 20 minutes and held at 40°C for two hours. The lyophilized product preferably has a final moisture content of less than about 5% and typically about 1 to 2%.

The lyophilizate prepared in accordance with the present invention can be reconstituted with water, saline, or another electrolyte or non-electrolyte diluent to give a colloidal dispersion for administraion in a conventional manner such as intravenous or intraperitoneal administration. The lyophilized product with the addition of water provides a colloidal dispersion of the lipid-porphyrin complex in an aqueous solution of the excipient. A colloidal dispersion consists of at least two discrete phases. The first is a dispersed or internal phase; the second is a continuous or external phase. Systems in the colloidal state contain one or more substances that have at least one dimension in the range of 10-100A to a few microns. See pp. 272-4 in Chapter 19, Disperse Systems, Remington's Pharmaceutical Sciences, 18th Edition, 1990, Mack Publishing Company, Easton, PA 18042. In the colloidal dispersions of the present invention, the dispersed or internal phase comprises particles of the porphyrin/lipid complex having a particle size in the range of 10 nm to 5000nm. In selecting the aqueous vehicle, it is recommended to use one having a specific gravity about equal to the lipid complex (est. 1.09 g/cc) to minimize the tendency for the dispersion to separate. The lyophilizate of the lipid complex can be reconstituted with water, saline, or another pharmaceutically acceptable aqueous diluent for intravenous administration. Upon reconstitution a dispersion is obtained which is suitable for injection. The lyophilizate can also be administered orally as an aqueous dispersion or as an encapsulated paste. While porphyrins are not generally administered subcutaneously because they cause necrosis, it has been observed that the lipid slows the release of the porphyrin into the tissue making it potentially feasible to administer the lipid-porphyrin complex subcutaneously. The preferred route of administration is intravenous or intrperitoneal.

For oral administration, the lyophilizate can be reconstituted to form an oral dispersion or formulated into a paste. Typically, the lyophilizate is filled into a soft gelatin capsule for oral administration.

Suitable dosages for lipid complexes of tin porphyrin range from about 10 to 200 mg/m²/hour. The drug is preferably administered as a continuous infusion over 0.5 to 5 hours using a programmable continuous infusion ambulatory pump or an IV infusion bag. After administration of the complex to the patient, the patient is exposed to radiation in photodynamic therapy in a known manner. This therapy is normally conducted by exposure to radiation in the range of 300 to 700 nm.

The invention will now be described in more detail with reference to the following non-limiting example.

### EXAMPLE

44.8 g of Cholesterol, 162.4 g of dimyristoyl phosphatidylcholine, 72.8 g of dimyristoyl phosphatidylglycol and 9.38 g of α-tocopherol were dissolved in 1,862 liters of chloroform. The purpose of d-tocopherol is to minimize oxidative damage to the molecule. Other anti-oxidants like BHT (butylated hydroxy toluene) or BHA (butylated hydroxy aninsole) may be used. 9.38 g of tin porphyrin (tin ethyl etioporphyrin) having the structure: 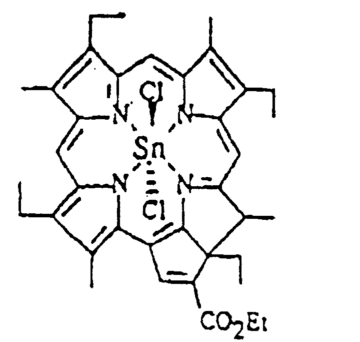 was added to the above solution and stirred for 15 minutes. A volume of water for injection, USP equal to the volume of chloroform used was added to the above solution and stirred vigorously for one hour. The resulting suspension was then sparged using nitrogen until all the chloroform was removed (less than 1000 pp of chloroform remained). To this suspension was added 348.6 g of mannitol dissolved in 3486 mL of water for injection, USP. The resulting product was homogenized using a Microfluidizer, Model 110Y. The resulting lipid/tin porphyrin complex formulation was filled in 50 cc amber, molded vials, 30 mL to a vial, and then freeze-dried.

The freeze-dried product was reconstituted in 20 mL of water for injection, USP. The reconstituted product was stable at room temperature for at least 144 hours, when protected from light.

The stability of the freeze-dried products was evaluated under varying conditions for up to 4 weeks. The results are shown in Table 1 where RS1, RS2, RS3, RS4 refer to HPLC peaks of products associated with degradation of the lyophilizate and "n.d." means "none detected."

**TABLE I**

| Stability Results: 1 day - 4 weeks | | | | | | |
|---|---|---|---|---|---|---|
| time | storage conditions | %of initial | RS1 | RS2 | RS3 | RS4 |
| 1 day | UV control | 99.75% | n.d. | n.d. | n.d. | n.d. |
| 1 day | UV | 101.90% | n.d. | n.d. | n.d. | n.d. |
| 1 day | 100 foot candles | 101.38% | n.d. | n.d. | n.d. | n.d. |
| 1 week | 50°C, 60% humidity | 100.03% | n.d. | n.d. | n.d. | n.d. |
| 2 weeks | 100 foot candles | 97.30% | n.d. | 0.21% | n.d. | n.d. |
| 2 weeks | 5°C | 100.93% | n.d. | n.d. | n.d. | n.d. |
| 2 weeks | 25°C, 60% humidity | 102.21% | n.d. | n.d. | n.d. | n.d. |
| 2 weeks | 40°C, 80% humidity | 100.52% | n.d. | n.d. | n.d. | n.d. |
| 2 weeks | 50°C, 60% humidity | 99.60 | 0.06% | n.d. | n.d. | n.d. |
| 3 weeks | 5°C | 100.52% | n.d. | n.d. | n.d. | n.d. |
| 3 weeks | 25°C, 60% humidity | 99.21% | n.d. | n.d. | n.d. | n.d. |
| 3 weeks | 40°C, 80% humidity | 97.02% | n.d. | n.d. | n.d. | n.d. |
| 3 weeks | 50°C, 60% humidity | 98.30% | n.d. | n.d. | 0.44% | n.d. |
| 4 weeks | 100 foot candles | 99.42% | n.d. | n.d. | n.d. | 0.34% |
| 4 weeks | 5°C | 100.68% | n.d. | n.d. | n.d. | n.d. |
| 4 weeks | 25°C, 60% humidity | 100.67% | n.d. | n.d. | n.d. | n.d. |
| 4 weeks | 40°C, 80% humidity | 98.65% | n.d. | n.d. | n.d. | 0.52% |
| 4 weeks | 50°C, 60% humidity | 99.16% | n.d. | n.d. | 0.84% | n.d. |

| RS | RRT* |
|---|---|
| 1 | .04 |
| 2 | 0.58 |
| 3 | 1.23 |
| 4 | 1.46 |

| | |
|---|---|
| * Relative Retention Time based on main sn Et₂ peak at 8.2 minutes | |

Having described the invention in detail and by reference to preferred embodiments thereof, it will be apparent that modifications and yariations are possible without departing from the scope of the invention defined in the appended claims.

## Claims

1. A method for preparing a lyophilizate of a lipid complex of a porphyrin which comprises the steps of preparing a concentrated solution of a porphyrin and a phospholipid in an organic solvent, adding water to the solution to cause formation of a complex of the porphyrin and the phopholipid, removing the organic solvent to provide a dispersion of the lipid complex in water as an aqueous phase, dissolving a pharmaceutically acceptable excipient in the aqueous phase of the dispersion, and lyophilizing the dispersion of the lipid complex to form a lyophilizate, wherein said porphyrin is a water insoluble metal porphyrin.

2. The method of claim 1 wherein said metal porphyrin is tin porphyrin.

3. The method of claim 2 wherein said tin porphyrin has the structure:

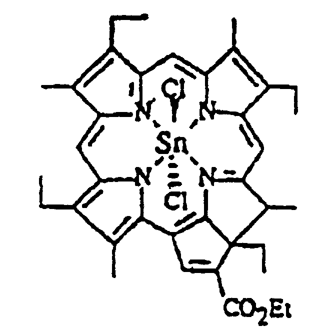

4. The method of claim 1 wherein said phospholipid is selected from the group consisting of dimyristoylphosphatidylcholine, dimyristoylphosphatidylglycerol, dipalmitoylphosphatidylcholine, dipalmitoylphosphatidylglycerol, distearoylphophatidylcholine, distearoylphosphatidylglycerol, and mixtures thereof.

5. The method of claim 4 wherein said phospholipid is a mixture of dimyristoylphosphatidylcholine and dimyristoylphosphatidylglycerol.

6. The method of claim 5 wherein said dimyristoylphosphatidylcholine is present in weight ratio to said dimyristoylphosphatidylglycerol of about 7 to 3.

7. The method of claim 1 wherein said lyophilization excipient is mannitol.

## Patentansprüche

1. Verfahren zum Herstellen eines Lipidkomplex-Lyophilisats eines Porphyrins, das die Schritte des Herstellens einer konzentrierten Lösung eines Porphyrins und eines Phospholipids in einem organischen Lösemittel, des Zugebens von Wasser zu der Lösung, um die Bildung eines Komplexes des Porphyrins und des Phospholipids herbeizuführen, des Entfernens des organischen Lösemittels, um eine Dispersion des Lipidkomplexes in Wasser als einer wäßrigen Phase bereitzustellen, des Lösens eines pharmazeutisch unbedenklichen Hilfsstoffs in der wäßrigen Phase der Dispersion und des Lyophilisierens der Dispersion des Lipidkomplexes, um ein Lyophilisat zu bilden, umfaßt, wobei das Porphyrin ein wasserunlösliches Metallporphyrin ist.

2. Verfahren nach Anspruch 1, wobei das Metallporphyrin Zinnporphyrin ist.

3. Verfahren nach Anspruch 2, wobei das Zinnporphyrin die folgende Struktur aufweist:

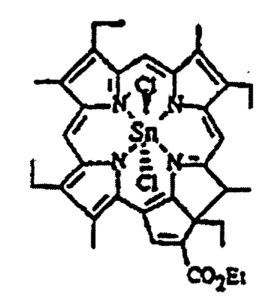

4. Verfahren nach Anspruch 1, wobei das Phospholipid aus der Gruppe ausgewählt ist, die aus Dimyristoylphosphatidylcholin, Dimyristoylphosphatidylglycerin, Dipalmitoylphosphatidylcholin, Dipalmitoylphosphatidylglycerin, Distearoylphosphatidylcholin, Distearoylphosphatidylglycerin und Mischungen davon besteht.

5. Verfahren nach Anspruch 4, wobei das Phospholipid eine Mischung aus Dimyristoylphosphatidylcholin und Dimyristoylphosphatidylglycerin ist.

6. Verfahren nach Anspruch 5, wobei das Dimyristoylphosphatidylcholin in einem Gewichtsverhältnis von etwa 7 zu 3 zu dem Dimyristoylphosphatidylglycerin vorliegt.

7. Verfahren nach Anspruch 1, wobei der Lyophilisationshilfsstoff Mannit ist.

## Revendications

1. Procédé selon la préparation d'un lyophilisat d'un complexe lipidique d'une porphyrine qui comprend les étapes de préparation d'une solution de concentré d'une porphyrine et d'un phospholipide dans un solvant organique, l'ajout d'eau à une solution en vue de provoquer la formation d'un complexe de porphyrine et de phospholipide, la suppression d'un solvant organique permettant de fournir une dispersion d'un complexe lipidique dans l'eau sous la forme d'une phase aqueuse, la dissolution d'un excipient acceptable d'un point de vue pharmaceutique dans une phase aqueuse de la dispersion et la lyophilisation de la dispersion du complexe lipidique pour former un lyophilisat, dans lequel ladite porphyrine est une porphyrine de métal non soluble dans l'eau.

2. Procédé selon la revendication 1 dans lequel ladite porphyrine métallique est la porphyrine étain.

3. Procédé selon la revendication 2 dans lequel ladite porphyrine étain présente la structure :

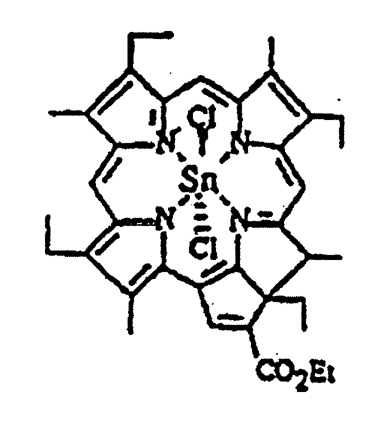

4. Procédé selon la revendication 1, dans lequel ledit phospholipide est sélectionné à partir du groupe se composant de la dimyristoyle phosphatidyle choline, du dimyristoyle phosphatidyle glycérol, de la dipalmitoyle phosphatidyle choline, du dipalmitoyle phosphatidyle glycérol, de la distéaroyle phosphatidyle choline, du distéaroyle phosphatidyle glycérol, et des mélanges de ceux-ci.

5. Procédé selon la revendication 4, dans lequel ledit phospholipide est un mélange de dimyristoyle phosphatidyle choline et de dimyristoyle phosphatidyle glycérol.

6. Procédé selon la revendication 5, dans lequel la dimyristoyle phosphatidyle choline est présente dans un rapport de poids par rapport au dit dimyristoyle phosphatidyle glycérol d'environ 7 pour 3.

7. Procédé selon la revendication 1, dans lequel ledit excipient de lyophilisation est le mannitol.
